## Europäisches Patentamt

## European Patent Office

## Office européen des brevets

(19)

(11) Veröffentlichungsnummer: **0 046 838**
**B1**

(12)

# EUROPÄISCHE PATENTSCHRIFT

(45) Veröffentlichungstag der Patentschrift:
24.06.87

(51) Int. Cl.⁴: **A 61 N 5/02**, H 03 L 5/02

(21) Anmeldenummer: **81101966.0**

(22) Anmeldetag: **17.03.81**

(54) Schaltungsanordnung zum Einstellen der Mikrowellenleistung von Mikrowellengeräten.

(30) Priorität: **13.08.80 DE 3030528**

(43) Veröffentlichungstag der Anmeldung:
**10.03.82 Patentblatt 82/10**

(45) Bekanntmachung des Hinweises auf die Patenterteilung:
**24.06.87 Patentblatt 87/26**

(84) Benannte Vertragsstaaten:
**AT BE CH DE FR GB IT LI LU NL SE**

(56) Entgegenhaltungen:
**DE - A - 1 954 260**
**DE - A - 2 929 947**
**FR - A - 925 960**
**FR - A - 2 220 924**

**MICROWAVE JOURNAL, Band 23, Nr. 1, Januar 1980, Seiten 39-44 Dedham, G.B. F. STERZER et al.:**
**"Microwave apparatus for the treatment of cancer"**

(73) Patentinhaber: **ROBERT BOSCH GMBH, Postfach 50, D-7000 Stuttgart 1 (DE)**

(72) Erfinder: **Böttcher, Lutz, Dipl.-Ing., Witzlebenstrasse 12, D-1000 Berlin 19 (DE)**

(74) Vertreter: **Schmidt, Hans-Ekhardt, Robert Bosch GmbH Geschäftsbereich Elektronik Patent- und Lizenzabteilung Forckenbeckstrasse 9-13, D-1000 Berlin 33 (DE)**

## Beschreibung

### Stand der Technik

Die Erfindung geht von einem Verfahren nach der Gattung des Hauptanspruchs aus.

Es ist schon bekannt (Microwave Journal, Vol. 23, Januar 1980, Seiten 39 bis 44), zur Einstellung der Mikrowellenleistung eines Mikrowellengerätes zwischen Mikrowellengenerator und Mikrowellenstrahler eine einstellbare Dämpfungsvorrichtung vorzusehen. Diese Art der Leistungseinstellung hat jedoch den Nachteil, dass die einstellbare Dämpfungsvorrichtung für eine verhältnismässig hohe Leistung ausgelegt sein muss.

### Aufgabe der Erfindung

Ausgehend von einem Verfahren gemäss der Gattung des Hauptanspruchs liegt der Erfindung die Aufgabe zugrunde, ein Verfahren anzugeben, mit dem eine Einstellung von Null bis zum Maximalwert der Mikrowellenleistung ohne ein Dämpfungsglied hoher Leistung möglich ist.

### Vorteile der Erfindung

Das erfindungsgemässe Verfahren mit den kennzeichnenden Merkmalen des Hauptanspruchs hat den Vorteil, dass die Mikrowellenleistung eines Mikrowellengerätes bei geringem Schaltungsaufwand sehr genau auf einen Wert zwischen Null und einem Maximalwert von zum Beispiel 100 W eingestellt werden kann. Es hat sich nämlich gezeigt, dass die Leistungsreflexionen beim Zusammenschalten eines Mikrowellengenerators und eines Strahlers bis zu einem gewissen Grade für den Generator unschädlich sind. Aus diesem Grunde kann man bei Mikrowellengeneratoren, die ein Stehwellenverhältnis von $S = \infty$ zulassen, auf die Anwendung von besonderen Entkopplungsmitteln, wie zum Beispiel einem Zirkulator und dem dann erforderlichen Abschlusswiderstand, verzichten. Bei einem endlichen Stehwellenverhältnis, zum Beispiel $S = 5$, kann durch Einschalten eines Dämpfungsgliedes zwischen Mikrowellengenerator und auf einer Fehlanpassung beruhendem Leistungssteller der gleiche einfache Aufbau gewählt werden.

Durch die in den Unteransprüchen aufgeführten Massnahmen sind vorteilhafte Weiterbildungen und Verbesserungen des im Hauptanspruch angegebenen Verfahrens möglich. Besonders vorteilhaft ist eine für das erfindungsgemässe Verfahren geeignete Schaltungsanordnung, bei der das Dämpfungsglied ein Leitungsstück mit definierter Dämpfung und einer Länge von mindestens $\lambda/2$ ist, wobei $\lambda$ die Betriebswellenlänge des Mikrowellengenerators auf der Leitung ist. Bei Verwendung eines derartigen Dämpfungsgliedes werden Störungseinflüsse auf den Generator durch die Einfügung des Dämpfungsgliedes in der Verbindungsleitung zwischen dem Mikrowellengenerator und der Vorrichtung zum Erzeugen einer einstellbaren Fehlanpassung vermieden.

### Zeichnung

Ein Ausführungsbeispiel der Erfindung ist in der Zeichnung anhand zweier Figuren dargestellt und in der nachfolgenden Beschreibung näher erläutert. Die Zeichnung zeigt in

Fig. 1 eine für das erfindungsgemässe Verfahren geeignete Schaltungsanordnung und

Fig. 2 einen Ausschnitt aus dem Blockschaltbild nach Fig. 1 mit einer Magnetfeldröhre, einem leitungsförmigen Dämpfungsglied und einer Vorrichtung zum Erzeugen einer einstellbaren Fehlanpassung.

### Beschreibung der Erfindung

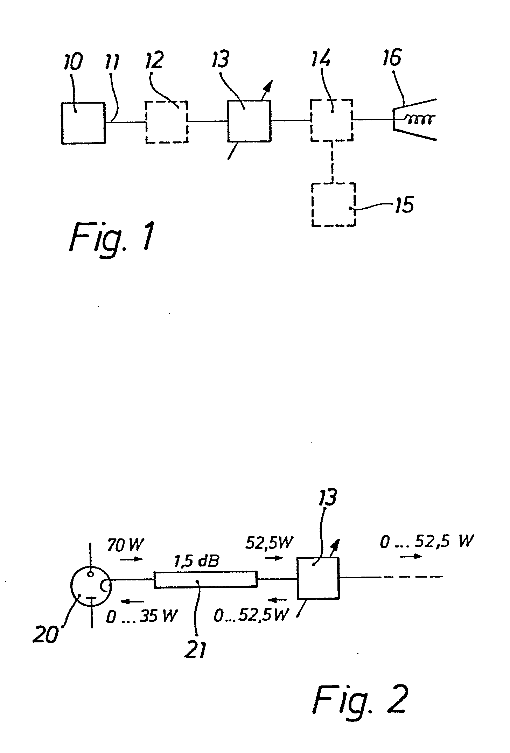

Nach der Fig. 1 weist eine Schaltungsanordnung zum erfindungsgemässen Einstellen der Mikrowellenleistung von Mikrowellengeräten einen Mikrowellengenerator 10 auf, dessen die Mikrowellenenergie abgebender Ausgang 11 über ein Dämpfungsglied 12 mit einer Vorrichtung 13 zum Erzeugen einer einstellbaren Fehlanpassung verbunden ist. An die Vorrichtung 13 schliesst sich über eine Weichenanordnung 14, mit der eine Leistungsmesseinrichtung 15 verbunden ist, ein Mikrowellenstrahler 16 an.

Das Dämpfungsglied 12, die Weichenanordnung 14 und die Leistungsmesseinrichtung 15 sind in Fig. 1 gestrichelt angedeutet, um zu zeigen, dass diese nicht zwingend vorzusehen sind. Beispielsweise kann auf das Dämpfungsglied 12 verzichtet werden, wenn für den Mikrowellengenerator 10 ein Stehwellenverhältnis von $S = \infty$ zugelassen ist.

Wenn das Einstellelement der Vorrichtung 13 zum Erzeugen einer einstellbaren Fehlanpassung mit einer geeichten Skala versehen ist, kann unter Umständen auch auf die Weichenanordnung 14 und die Leistungsmesseinrichtung 15 verzichtet werden.

Für den Fall, dass ein Stehwellenverhältnis von $S = \infty$ nicht zugelassen werden darf, um eine zu dem Mikrowellengenerator 10 gehörende Magnetfeldröhre 20 (vgl. Fig. 2) nicht zu überlasten, ist ein Dämpfungsglied 21 erforderlich. Dieses Dämpfungsglied hat zum Beispiel bei 70 W Generatorleistung und einem zulässigen Stehwellenverhältnis von $S = 6$ eine Dämpfung von etwa 1,5 dB. Unter diesen Umständen beträgt die Mikrowellenleistung nach dem Passieren des Dämpfungsgliedes 21 etwa 52,5 W, die bei hundertprozentiger Fehlanpassung durch die Vorrichtung 13 vollständig reflektiert wird und nach dem zweiten Passieren des Dämpfungsgliedes 21 auf etwa 35 W reduziert wird. Auf diese Weise wird das zulässige Stehwellenverhältnis von $S = 6$ nicht überschritten. Das Dämpfungsglied besteht aus einem Leitungsstück mit dem erforderlichen bestimmten Dämpfungswert und mit einer Länge von mindestens $\lambda/2$. Dabei bedeutet $\lambda$ die Betriebswellenlänge des Mikrowellengenerators in dem das Dämpfungsglied bildenden Leitungsstück.

### Patentansprüche

1. Verfahren zum Einstellen der Mikrowellenleistung von Mikrowellengeräten, vorzugsweise Mikrowellen-Bestrahlungsgeräten, mit einem Mikrowellengenerator (10) und einem Mikrowellenstrahler (16) sowie einer Reihenschaltung aus einem Dämpfungsglied (12) und einer Anpassungsvorrichtung (13) zwischen dem Mikrowellengenerator und dem Mikrowellenstrahler, dadurch gekennzeichnet, dass die Mikrowellenleistung auf einen unter einem vorgegebenen Maximalwert liegenden Leistungswert bei fest eingestelltem Dämpfungsglied über eine vorgegebene Fehlanpassung gesteuert wird.

2. Schaltungsanordnung für ein Verfahren nach Anspruch 1, dadurch gekennzeichnet, dass zum Erzeugen einer einstellbaren Fehlanpassung eine Kurzschlussleitung einstellbarer Länge vorgesehen ist.

3. Schaltungsanordnung für ein Verfahren nach Anspruch 1, dadurch gekennzeichnet, dass das Dämpfungsglied (21) ein Leitungsstück mit definiertem Dämpfungswert und einer Länge von mindestens $\lambda/2$ ist, wobei $\lambda$ die Betriebswellenlänge des Mikrowellengenerators (10) in dem das Dämpfungsglied bildenden Leitungsstück ist.

4. Schaltungsanordnung für ein Verfahren nach Anspruch 1 oder 3, dadurch gekennzeichnet, dass der Mikrowellengenerator (10) eine Magnetfeldröhre (20) aufweist.

## Claims

1. A method for adjusting the microwave power of microwave apparata, preferably microwave radiation apparata, having a microwave generator (10) and a microwave radiator (16) as well as a series connection comprising an attenuator (12) and a matching device (13) between the microwave generator and the microwave radiator, characterised in that the microwave power is regulated to a value lying below a predetermined maximum value by way of a predetermined mismatch, the attenuator being fixedly adjusted.

2. A circuit arrangement for a method as claimed in Claim 1, characterised in that a short-circuit lead of adjustable length is provided to generate an adjustable mismatch.

3. A switching arrangement for a method as claimed in Claim 1, characterised in that the attenuator (21) is a piece of conductor having a defined attenuation value and a length of at least $\lambda/2$, $\lambda$ being the operating wavelength of the microwave generator (10) in the piece of conductor forming the attenuator.

4. A switching arrangement for a method as claimed in Claim 1 or 3, characterised in that the microwave generator (10) has a magnetron (20).

## Revendications

1. Procédé pour régler la puissance micro-ondes d'appareils micro-ondes, de préférence d'appareils pour l'irradiation par micro-ondes, avec un générateur de micro-ondes (10) et un émetteur de rayonnement micro-ondes (16), ainsi qu'avec un montage en série d'un organe d'atténuation (12) et d'un dispositif d'adaptation (13) entre le générateur de micro-ondes et l'émetteur de rayonnement micro-ondes, procédé caractérisé en ce que la puissance micro-ondes est commandée à une valeur de puissance se situant au-dessous d'une valeur maximale prédéfinie, pour un organe d'atténuation réglé de façon fixe, par l'intermédiaire d'une désadaptation prédéfinie.

2. Dispositif de circuit pour la mise en œuvre d'un procédé selon la revendication 1, caractérisé en ce qu'il est prévu, pour obtenir une désadaptation réglable, une liaison de court-circuit de longueur réglable.

3. Dispositif de circuit pour la mise en œuvre d'un procédé selon la revendication 1, caractérisé en ce que l'organe d'atténuation (21) est une pièce de liaison avec une valeur d'atténuation définie et une longueur d'au moins $\lambda/2$, étant la longueur d'onde de fonctionnement du générateur de micro-ondes (10) dans lequel est la pièce de liaison constituant l'organe d'atténuation.

4. Dispositif de circuit pour la mise en œuvre d'un procédé selon la revendication 1 ou 3, caractérisé en ce que le générateur de micro-ondes (10) comporte un magnétron à ondes progressives (20).

*Fig. 1*

*Fig. 2*